# EUROPEAN PATENT APPLICATION

(11) **EP 3 470 391 A1**
(43) Date of publication of application: **17.04.2019**
(21) Application number: 17810353.7
(22) Date of filing: 07.06.2017
(51) Int. Cl.: C07C 235/06, C07C 235/14, A61K 8/42, A61Q 1/00, A61Q 19/00, B01F 17/22, C09K 3/00

(54) **COSMETIC BASE INCLUDING AMIDE ALCOHOL ESTER, AND COSMETIC**

(30) Priority: 08.06.2016 JP 2016114277
(71) Applicant: Kokyu Alcohol Kogyo Co., Ltd., Narita-shi, Chiba 287-0225 (JP)
(72) Inventor: INOUE, Takanori, Narita-shi Chiba 287-0225 (JP); MASUNO, Mari, Narita-shi Chiba 287-0225 (JP)
(74) Representative: Cabinet Nuss
(86) International application number: PCT/JP2017/021160
(87) International publication number: WO 2017/213179

(57) **Abstract**

The purpose of the present invention is to provide a novel amide alcohol ester that can be used as a cosmetic base ingredient. An amide alcohol ester represented by formula (I).

## Description

### [Technical Field]

The present invention relates to a cosmetic base and a cosmetic product comprising an ester of amide alcohol.

### [Background Art]

Conventionally, various ester compounds have been known as a cosmetic base component used in the production of cosmetic products. Representative cosmetic base components include esters of alcohol and fatty acid, and ester compounds composed of atoms of carbon, hydrogen and oxygen are common.

Meanwhile, nitrogen-containing esters are not so used as a cosmetic base due to problems such as odor derived from nitrogen atoms; for example, an amino acid ester is known as a gelling agent for an oil agent (Patent Document 1), and an N-long chain acyl acidic amino acid ester is known as a compound having emollient providing power and emulsifying power (Patent Document 2). This amino acid ester is susceptible to hydrolysis, and there are problems of odor and deterioration of quality due to heating and aging. In order to prevent decomposition and prolong the quality, refrigeration is necessary, and consideration must be given to the storage method.

### [Citation List]

### [Patent Document]

[Patent Document 1] JP A 2002-316971
[Patent Document 2] JP A H07-118290

### [Disclosure of Invention]

### [Problems to Be Solved by the Invention]

A novel nitrogen-containing ester compound suitable for use in a cosmetic product, which can solve the problem of odor and deterioration of quality and the like due to heating and aging of conventional amino acid esters, is provided.

### [Means for Solving the Problems]

The inventors of the present invention have discovered that, in their strenuous research to solve the above problems, a novel ester of amide alcohol has various excellent properties as a cosmetic base component, and as a result of further research, the present inventors have completed the present invention.

That is, the present invention relates to the following [1] to [9].
[1] A compound represented by formula (I): 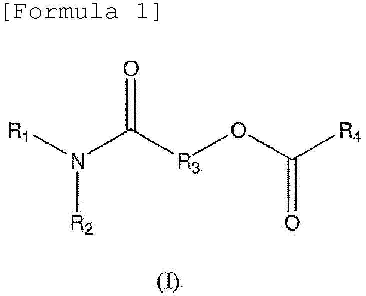 wherein
   R₁ is an optionally substituted C6-C22 hydrocarbon group,
   R₂ is H, or an optionally substituted C6-C22 hydrocarbon group,
   R₃ is an optionally substituted linear or branched C2-C21 hydrocarbon group,
   R₄ is an optionally substituted C5-C42 hydrocarbon group; or a group represented by formula (II) below: 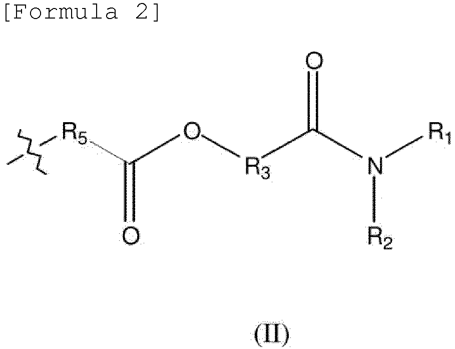
   wherein in formula (II),
   R₁ to R₃ are the same as defined above,
   R₅ is an optionally substituted C2-C42 hydrocarbon group.
[2] A thickener or a gelling agent comprising the compound according to [1].
[3] A powder dispersant comprising the compound according to [1].
[4] A texture improving agent comprising the compound according to [1].
[5] A cosmetic product comprising the compound according to [1] .
[6] The cosmetic product according to [5], comprising an oil agent.
[7] The cosmetic product according to [5] or [6], comprising a powder.
[8] The cosmetic product according to [5] or [6], which is for cleansing.
[9] The cosmetic product according to any one of [5] to [7], which is for make-up.

### [Advantageous Effects of Invention]

The ester of amide alcohol of the present invention has either no odor, or very little odor if any. In addition, the ester of amide alcohol is stable, there is no problem such as generation of offensive smell due to deterioration over time, and it is a nitrogen-containing compound suitable for use in cosmetic products.

The esters of amide alcohols of the present invention have various forms such as solid, semi-solid, and liquid, and can be used for various cosmetic products by utilizing their respective properties.

### [Brief Description of Drawings]

Figure 1 is a diagram showing evaluation results of rough feeling on the hair of esters of liquid amide alcohols and various oil agents.
Figure 2 is a diagram showing the zinc oxide-dispersibility of esters of amide alcohols and various oil agents.
Figure 3 is a diagram showing the red iron oxide-dispersibility of esters of amide alcohols and various oil agents.

### [Description of Embodiments]

The compound of the present invention is represented by formula (I) below: 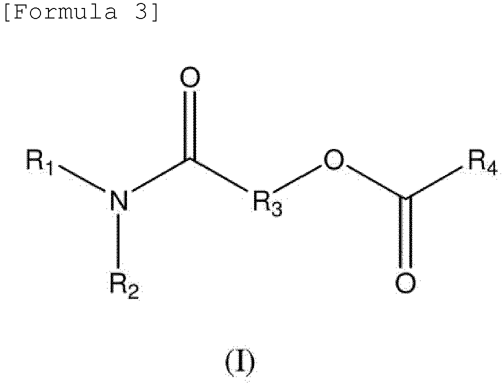 wherein
R₁ is an optionally substituted C6-C22 hydrocarbon group,
R₂ is H, or an optionally substituted C6-C22 hydrocarbon group,
R₃ is an optionally substituted linear or branched C2-C21 hydrocarbon group,
R₄ is an optionally substituted C5-C29 hydrocarbon group; or a group represented by formula (II) below: 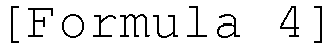 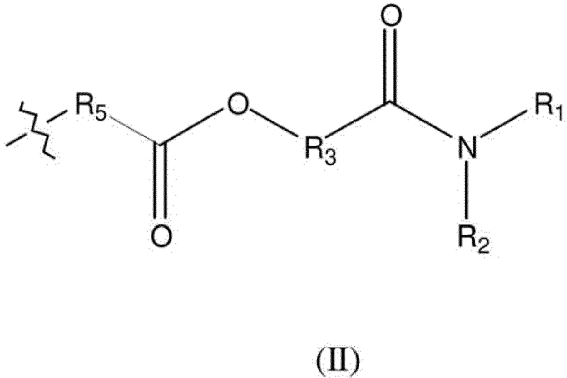
wherein in formula (II),
R₁ to R₃ are the same as defined above,
R₅ is an optionally substituted C2-C42 hydrocarbon group.

As used herein, the term "hydrocarbon group" may be, unless otherwise specified, saturated or unsaturated, linear, branched or cyclic, or a combination of linear or branched with cyclic, and includes a hydrocarbon group consisting of a linear or branched hydrocarbon moiety such as benzyl group, phenylethyl group, etc. and a cyclic hydrocarbon moiety.

That is, the C6-C22 hydrocarbon group in R₁ and R₂ includes a linear, branched or cyclic C6-C22 hydrocarbon group, or a C6-C22 hydrocarbon group consisting of a linear or branched hydrocarbon moiety and a cyclic hydrocarbon moiety, and examples thereof include cyclic groups such as cyclohexyl, decahydronaphthyl, tetrahydrodicyclopentadiene, sterol, phenyl, naphthyl, anthracenyl, etc.; branched alkyl groups such as ethylhexyl, isostearyl, octyldodecyl, etc.; multibranched alkyl groups such as dimethyl, trimethyl , tetramethyl, etc.; linear alkyl groups such as hexyl, octyl, lauryl, myristyl, cetyl, stearyl, arachyl, behenyl, etc.; and alkenyl groups such as oleyl and elaidyl, etc.

In one embodiment of the invention, R₁ is preferably cyclohexyl, ethylhexyl, octyl, lauryl, myristyl, stearyl, oleyl, benzyl or phenylethyl.

In one embodiment of the present invention, R₂ is preferably H.

The hydrocarbon group in R₃ is a linear or branched C2-C21 hydrocarbon group having no cyclic structure, and examples thereof include alkyl groups such as propyl, butyl, pentyl, hexyl, heptyl, octyl, ethylhexyl, etc., and alkenyl groups such as butylene, pentylene, hexylene, heptylene, etc.

In one embodiment of the present invention, R₃ is preferably propylene, butylene, pentylene or hexylene.

The C5-C29 hydrocarbon group in R₄ comprises a linear, branched or cyclic C5-C29 hydrocarbon group, or a C5-C29 hydrocarbon group consisting of a linear or branched hydrocarbon moiety and a cyclic hydrocarbon moiety, and is typically a residue of fatty acid; examples thereof include cyclic groups such as cyclohexyl, decahydronaphthyl, tetrahydrodicyclopentadiene, sterol, phenyl, naphthyl, anthracenyl, etc.; branched alkyl groups such as ethylhexyl, isostearyl, octyldodecyl, etc.; multibranched alkyl groups such as dimethyl, trimethyl , tetramethyl, etc.; linear alkyl groups such as hexyl, octyl, lauryl, myristyl, cetyl, stearyl, arachyl, behenyl, etc.; alkenyl groups such as oleyl and elaidyl, etc.

In the group represented by formula (II) in R₄: 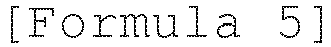 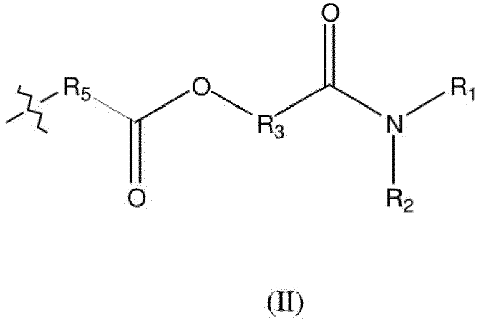 R₁ to R₃, respectively, may be the same as or different from R₁ to R₃ in formula (I), but preferably they are the same, and the compound wherein R₁ to R₃ are the same as those in formula (I) can be represented by the following formula:

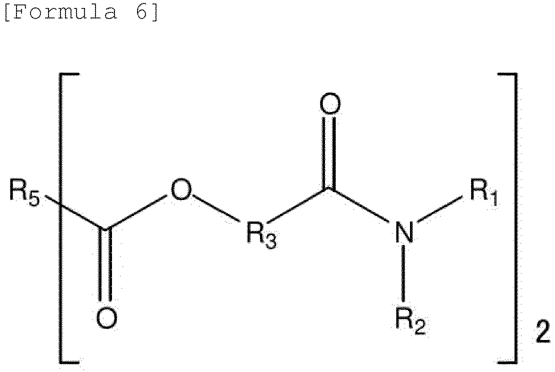

The hydrocarbon group in R₅ comprises a linear, branched or cyclic C2-C42 hydrocarbon group, or a C2-C42 hydrocarbon group consisting of a linear or branched hydrocarbon moiety and a cyclic hydrocarbon moiety, and is typically a residue of carboxylic acid; examples thereof include alkylene groups such as ethyl, butyl, hexylene, octyl, hydrogenated dimer dilinoleate, etc., and alkenylene groups such as butene and hexene.

In the present invention, each hydrocarbon group may be substituted, and may be substituted with, for example, a hydroxy group, a carboxy group, an aldehyde group.

Examples of substituted C6-C22 hydrocarbon group in R₁ and R₂ include hexanol, ethylcyclohexanol, hexanoic acid.

Examples of substituted C2-C21 hydrocarbon group in R₃ include hydroxybutyl, butyl ketone.

Examples of a substituted C5-C29 hydrocarbon group in R₄ include hydroxylauryl, hydroxyisostearyl.

Examples of a substituted C2-C42 hydrocarbon group in R₅ include hydroxyethyl, hydroxy hydrogenated dimer dilinoleate.

In a particular embodiment of the invention, R₅ is a residue of dicarboxylic acid, in particular a residue of dimer acid.

Dimer acid is a dibasic acid having a carbon number of 36, obtained by two molecule polymerization of Diels Alder reaction of unsaturated fatty acid having a carbon number of 18 such as oleic acid, linoleic acid, etc. A saturated aliphatic dibasic acid having a carbon number of 36 generated by reduction of a double bond in dimer acid by hydrogenation reduction is also called hydrogenated dimer acid. Dimer acid is usually produced using an unsaturated fatty acid having a carbon number of 18 mainly composed of oleic acid or linoleic acid, and is obtained as a mixture consisting of many compounds including a monomer acid, a trimer acid, etc. obtained as by-products. The dimer acid used in the present invention may be a mixture of two or more kinds obtained from such a mixture. In one embodiment of the present invention, the dimer acid is preferably a hydrogenated dimer acid, and it is preferable that double bonds of the dimer acid are completely hydrogenated, but it is also acceptable that a part of the double bonds remains.

### <Method for producing compound of formula (I)>

An ester of amide alcohol of formula (I) can be produced using known ester production methods.

For example, it can be produced by esterification reaction in a conventional manner of an amide alcohol represented by formula (III) : 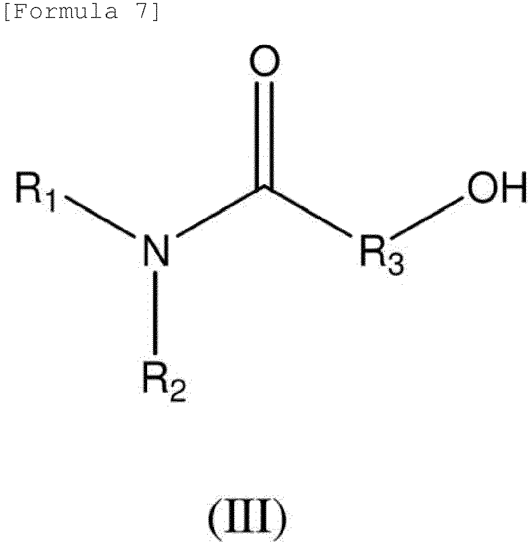 wherein
R₁ to R₃ are the same as those in formula (I),
with an optionally substituted C6-C30 fatty acid or an optionally substituted C4-C44 dicarboxylic acid.

In one embodiment of the present invention, examples of fatty acid used in the synthesis of the compound of formula (I) include lauric acid, myristic acid, isostearic acid, etc.

In one embodiment of the present invention, examples of dicarboxylic acid to be used include succinic acid, adipic acid, hydrogenated dimer dilinoleic acid, behenic acid dimer, etc.

### <Cosmetic base>

The present invention provides a cosmetic base comprising the compound represented by formula (I).

In the present specification, the term "cosmetic base" refers to a component (base ingredient) that provides basic shape and performance in a cosmetic product, for example, a solid fat, a liquid oil, a gelling agent, a thickener, etc., but it is not limited thereto.

In the present specification, "cosmetic base" may consist exclusively of the compound of formula (I), or may comprise other cosmetic base components.

In one embodiment of the present invention, the cosmetic base is a composition comprising, in addition to the compound of formula (I), other cosmetic base components usually used to provide a specific shape to cosmetic products.

Other cosmetic base components include, but are not limited to, solid fats and oils, liquid oils, gelling agents, thickeners and silicone oils, silicone derivatives, surfactants, water, etc., other than the compounds of formula (I).

The compounds of formula (I) and cosmetic bases comprising them can be used for the production of various cosmetic products.

In the present specification, the term "cosmetic product" means any product that is applied to the skin, hair, lips, etc. for the purpose of cleaning the body and beautifying the appearance, although not particularly limited thereto. Specific examples include, but are not limited to, skin care products, makeup products, hair care products, body care products, etc.

Examples of skin care products include, but are not limited to, lotion, cream, milky lotion, gel, beauty essence, cosmetic oil, pack, cleansing, face cleanser, whitening cosmetics, UV care cosmetics, etc.

Examples of makeup products include, but are not limited to, makeup base, foundation, lip color, lipstick, lip cream, lip gloss, cheek color, eyeliner, mascara, eye shadow, eyebrow, etc.

Examples of hair care products include, but are not limited to, shampoo, conditioner, hair rinse, hair treatment, hair styling agent, permanents, hair color, etc.

Examples of body care products include, but are not limited to, body shampoo, body lotion, hand cream, nail cream, deodorant cosmetics, etc.

The compound of formula (I) is suitable for use in cosmetic products comprising an oil agent, such as cleansing oils, hair treatments and skin creams, because of its excellent compatibility with oil agents.

In addition, the compound of formula (I) is suitable for use in semisolid or solid cosmetic products comprising an oil agent, since it has the ability to thicken and gelate oil agents. In one embodiment of the present invention, the compound of formula (I) can also be used as a thickener/gelling agent for cosmetic products.

Since the compound of formula (I) has various forms such as solid, semi-solid and liquid form at room temperature, it can be utilized in various cosmetic products utilizing such property.

In one embodiment of the present invention, an ester of amide alcohol that is in liquid state at room temperature is excellent in powder dispersibility of pigments such as titanium oxide, zinc oxide, iron oxide, etc. even though its viscosity is high. That is, it is superior in powder dispersibility to the ester oils having the same viscosity. Therefore, the compound of formula (I) can be used as a powder dispersant, and specifically, it is suitable for use as a base for powder-containing cosmetics (for example, UV care products, make-up products such as lipsticks, foundations, cheeks and eye shadows, etc.), and as a base for cleansing products.

In one embodiment of the present invention, an ester of amide alcohol that is solid at room temperature has a gelling ability with an ester or a hydrocarbon oil. In addition, since the melting point is in the range of 40 to 80°C, handling at the time of production is favorable, and there is no problem such as difficulty in melting, for example in the production in a large pot. Furthermore, with respect to esters of amide alcohols having a melting point in the vicinity of 40°C, since they melt at about body temperature, they have a texture of melting at the time of application, and are suitable for application to cosmetics relating to skin care products and make-up products (especially lip products).

The shape of cosmetic product is not particularly limited, and it may be solid, liquid, milky lotion, cream, gel and the like. Those skilled in the art can appropriately select an amide alcohol ester suitable for a desired shape.

The blending amount of the compound of formula (I) in a cosmetic product varies depending on the type of desired cosmetic product and other materials to be combined, and it can be appropriately adjusted by those skilled in the art.

Cosmetic products can be produced by a known cosmetic production method. For example, it can be obtained by dissolving a cosmetic base component under stirring to obtain a homogeneous mixture, then adding an additive such as a perfume, and molding. In another embodiment of the present invention, it is also possible to disperse a powder such as a pigment in the compound of formula (I) and then mix it with other cosmetic base components to obtain a homogeneous compound, followed by molding.

In one embodiment of the present invention, there is provided a method for producing a cosmetic product, comprising adding an ester of amide alcohol of the present invention.

In one embodiment of the present invention, there is provided a method for producing a cosmetic product, comprising adding an ester of amide alcohol of the present invention to thicken or gelate.

In one embodiment of the present invention, there is provided a method for producing a cosmetic product, comprising adding an ester of amide alcohol of the present invention to disperse a powder such as a pigment.

In one embodiment of the present invention, it is possible to improve the texture of cosmetic products by adding the ester of amide alcohol of the present invention. In a preferred embodiment of the present invention, a cosmetic product is provided with a smooth texture (feeling of use/feeling of application) by adding the ester of amide alcohol of the present invention.

Accordingly, in one embodiment of the present invention, there is provided a method for improving/ameliorating the texture of cosmetic products comprising adding the ester of amide alcohol of the present invention.

The blending amount of the compound of formula (I) in a cosmetic product varies depending on the type of desired cosmetic product and other materials to be combined, and it can be appropriately adjusted by those skilled in the art. Meanwhile, in the present invention, unless especially stated clearly, % means weight %.

### [Example]

### [Synthesis example 1] CHBA-L Synthesis of N-cyclohexylbutyramide-4-lauric acid ester

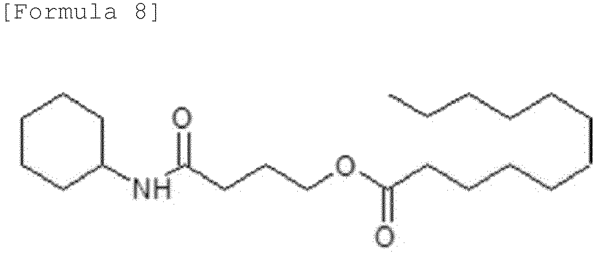

235 g of 4-hydroxy-N-cyclohexyl, butyramide and a sufficient amount of methyl laurate were dissolved in toluene, NaOMe as a catalyst was added thereto, and the mixture was heated under reflux at 120°C for 4 hours and 257 g (yield 66%) of a white solid was obtained by transesterification reaction. (m.p. = 80°C)

After the synthesis, purification was carried out by washing with water, drying and deodorization to obtain an ester having a purity of 99%.

### [Synthesis example 2] EHBA-L

### Synthesis of N-2 ethylhexylbutyramide-4-lauric acid ester

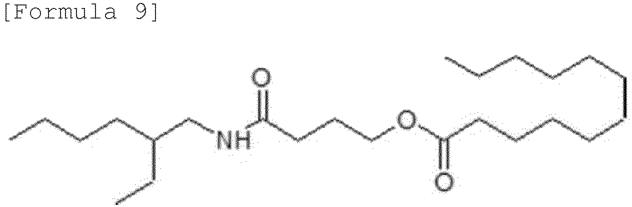

75 g of 4-hydroxy-N-2-ethylhexyl, butyramide and a sufficient amount of methyl laurate were dissolved in toluene, NaOMe as a catalyst was added thereto, and the mixture was heated under reflux at 130°C for 8 hours and 125 g (yield 90%) of a milky white solid was obtained by conventional esterification reaction. (Melted on human skin. Predicted m.p. = 32°C)

After the synthesis, purification was carried out by washing with water, drying and deodorization to obtain an ester having a purity of 97%.

### [Synthesis example 3] OBA-L

### Synthesis of N-octylbutyramide-4-lauric acid ester

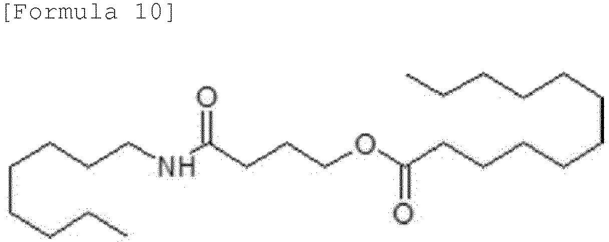

111 g of 4-hydroxy-N-octyl, butyramide and a sufficient amount of methyl laurate were dissolved in toluene, NaOMe as a catalyst was added thereto, and the mixture was heated under reflux at 120°C for 8 hours and 186 g (yield 90%) of a milky white solid was obtained by conventional esterification reaction. (m.p. = 68°C)

After the synthesis, purification was carried out by washing with water, drying and deodorization to obtain an ester having a purity of 92%.

### [Synthesis example 4] LBA-L

### Synthesis of N-laurylbutyramide-4-lauric acid ester

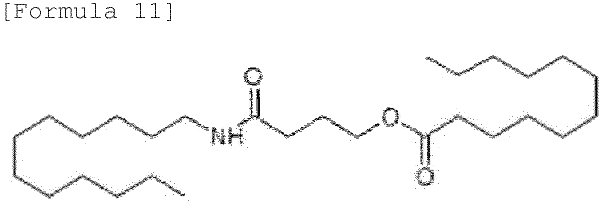

126 g of 4-hydroxy-N-lauryl, butyramide and a sufficient amount of methyl laurate were dissolved in toluene, NaOMe as a catalyst was added thereto, and the mixture was heated under reflux at 110°C for 3 hours and 154 g (yield 78%) of a milky white solid was obtained by conventional esterification reaction. (m.p. = 70°C)

After the synthesis, purification was carried out by washing with water, drying and deodorization to obtain an ester having a purity of 95%.

### [Synthesis example 5] CHBA-IS

### Synthesis of N-cyclohexylbutyramide-4-isostearic acid ester

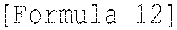

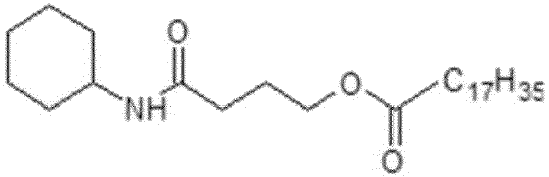

The reaction was carried out in the same manner as in Synthesis example 1 except that methyl laurate of Synthesis example 1 was changed to methyl isostearate, thereby obtaining 468 g (yield 82%) of a yellow liquid. (Viscosity: 230 mPa·s (25°C))

After the synthesis, purification was carried out by washing with water, drying and deodorization to obtain an ester having a purity of 99%.

### [Synthesis example 6] EHBA-IS

### Synthesis of N-2 ethylhexylbutyramide-4-isostearic acid ester

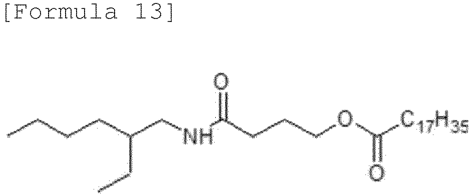

The reaction was carried out in the same manner as in Synthesis example 2, except that methyl laurate in Synthesis example 2 was changed to methyl isostearate, thereby obtaining 277 g (yield 73%) of a yellow liquid. (Viscosity: 270 mPa·s (25°C))

After the synthesis, purification was carried out by washing with water, drying and deodorization to obtain an ester having a purity of 99%.

### [Synthesis example 7] OBA-IS

### Synthesis of N-octylbutyramide-4-isostearic acid ester

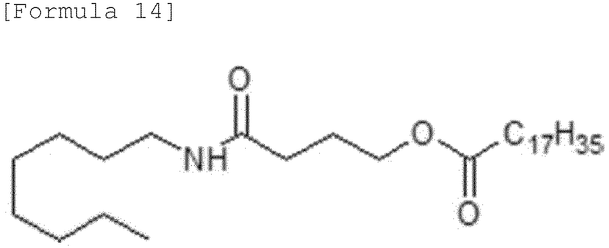

The reaction was carried out in the same manner as in Synthesis example 3 except that methyl laurate in Synthesis example 3 was changed to methyl isostearate, thereby obtaining 295 g (yield 74%) of a milky white solid. (Melted on human skin. Predicted m.p. = 31°C)

After the synthesis, purification was carried out by washing with water, drying and deodorization to obtain an ester having a purity of 99%. The ester is thixotropic.

### [Synthesis example 8] LBA-M

### Synthesis of N-laurylbutyramide-4-myristic acid ester

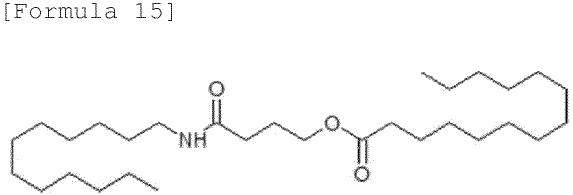

The reaction was carried out in the same manner as in Synthesis example 4 except that methyl laurate in Synthesis example 4 was changed to methyl isostearate, thereby obtaining 117 g (yield 82%) of a milky white solid. (m.p. = 72°C)

After the synthesis, purification was carried out by washing with water, drying and deodorization to obtain an ester having a purity of 95%.

### [Synthesis example 9] STBA-IS

### Synthesis of N-stearylbutyramide-4-isostearic acid ester

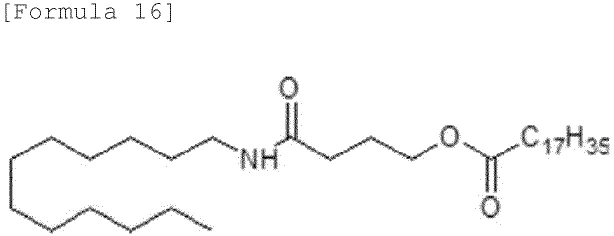

71 g of 4-hydroxy-N-stearyl, butyramide and a sufficient amount of methyl stearate were dissolved in toluene, NaOMe as a catalyst was added thereto, and the mixture was heated under reflux at 110°C for 4 hours and 97 g (yield 79%) of a milky white solid was obtained by transesterification reaction. (m.p. = 47°C)

After the synthesis, purification was carried out by washing with water, drying and deodorization to obtain an ester having a purity of 92%.

### [Synthesis example 10] 2CHBA-D

### Synthesis of N-dicyclohexylbutyramide dimer acid ester

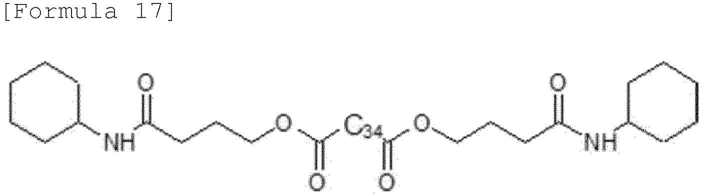

Using dimethyl dimerate (1.00 equivalent, AV: 1.1), 4-hydroxy-N-cyclohexyl, butyramide (2.10 equivalents) and NaOMe (0.05 equivalent) as ingredients, the reaction was carried out at 150°C for 2 hours, then washing with water, drying (120°C, 1 hour), and deodorization (150°C, 4 hours) were carried out to obtain a brown viscous liquid (yield: 85.2%, viscosity: 10300 mPa·s (25°C)).

The obtained viscous liquid comprised 56.8% of dimeric body (dicyclohexyl dimerate, butyramide), 39.6% of monomeric body, and 3.6% of dimethyl.

Physical property values of the esters of amide alcohols obtained in Synthesis examples 1 to 8 are shown below.

In the present specification, each ester of amide alcohol is represented by using the following abbreviations.
Synthesis example 1: "CHBA-L" (N-cyclohexylbutyramide-4-lauric acid ester)
Synthesis example 2: "EHBA-L" (N-2 ethylhexylbutyramide-4-lauric acid ester)
Synthesis example 3: "OBA-L" (N-octylbutyramide-4-lauric acid ester)
Synthetic Example 4 "LBA-L" (N-laurylbutyramide-4-lauric acid ester)
Synthesis example 5: "CHBA-IS" (N-cyclohexylbutyramide-4-isostearic acid ester)
Synthesis example 6: "EHBA-IS" (N-2 ethylhexylbutyramide-4-isostearic acid ester)
Synthesis example 7: "OBA-IS" (N-octylbutyramide-4-isostearic acid ester)
Synthesis example 8: "LBA-M" (N-laurylbutyramide-4-myristic acid ester)

### <Gelling ability>

The amide alcohol or ester of amide alcohol was heated and mixed with each liquid, and the critical gelation concentration was measured by sample tube inversion method.
Sample tube used: used tube: Maruemu screw tube No. 7 (trunk diameter 35 mm, total length 78 mm)

### [Table 1]

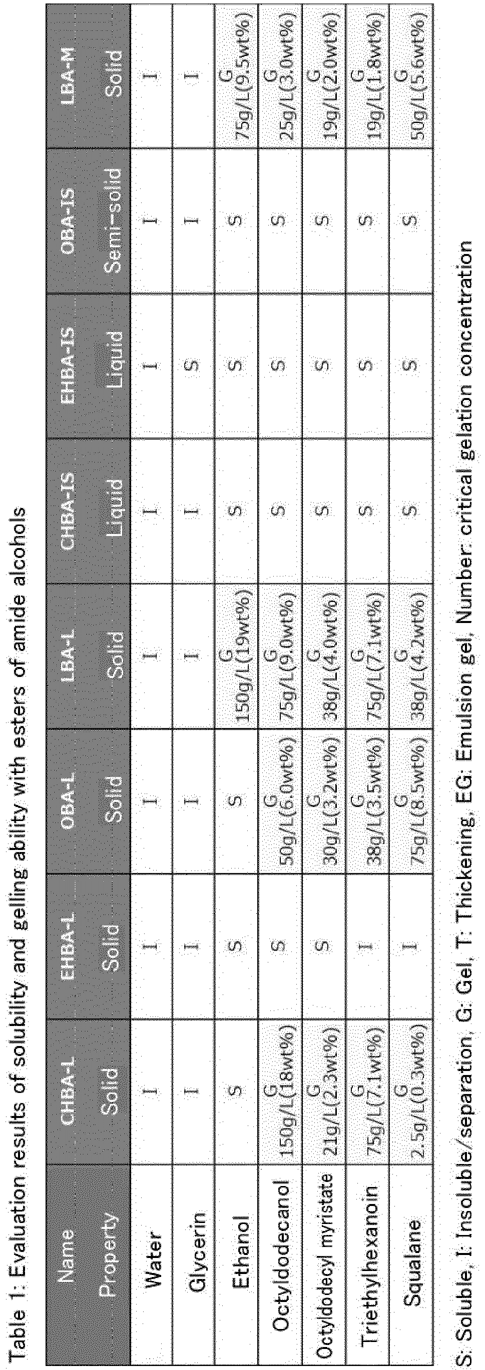

It was confirmed that the esters of amide alcohols of the present invention as a whole have good solubility with oily components, and that many esters of amide alcohols have gelling ability.

As a conventional gelling agent for gelling an oil agent such as esters, 12-hydroxystearic acid, a polyamide resin, an amino acid type gelling agent can be mentioned; as a conventional thickener, a dextrin fatty acid ester, etc. can be mentioned.

The polyamide resin is excellent in gelling ability with a polar ester, but its gelling ability with a nonpolar ester or a hydrocarbon oil tends to be inferior, so that the kind of oil agent to be gelled is limited. With respect to amino acid type gelling agents, they gelate polar and non-polar oil agents widely; however, even when only a small amount of amino acid type gelling agent is used, the obtained gelled product is relatively hard and brittle (crack) may be seen. In addition, many polyamide resins and amino acid type gelling agents have high melting point, and they are liable to be concerned in terms of facility problems during the production.

Meanwhile, since the melting point of esters of amide alcohols is 40 to 80°C, they are easy to dissolve and have an advantage. In addition, an liquid oil thickened or gelled with an ester of amide alcohol is comparatively soft, and it can be "gelled" or "thickened" by increasing or decreasing the concentration of the ester of amide alcohol; accordingly, improvement of the texture of the liquid oil in accordance with the purpose is possible.

Human hair strand BS-B3N (100% black hair, root aligning II, Beaulax Co., Ltd.) was damage-treated by the method prescribed by our company, to make damaged hair.
Approximately 0.2 mL of an oil agent was added dropwise to the dry damaged hair, and uniformly applied using a spatula.
100 damaged hairs were arranged in a width of 1 cm as evenly as possible, and MMD was measured using friction tester KES-SE (Kato Tech Co., Ltd.).

MMD: Variable value of friction coefficient. As the MMD value decreases, fluctuation of friction coefficient is small and roughness is small, so the smoothness is good.

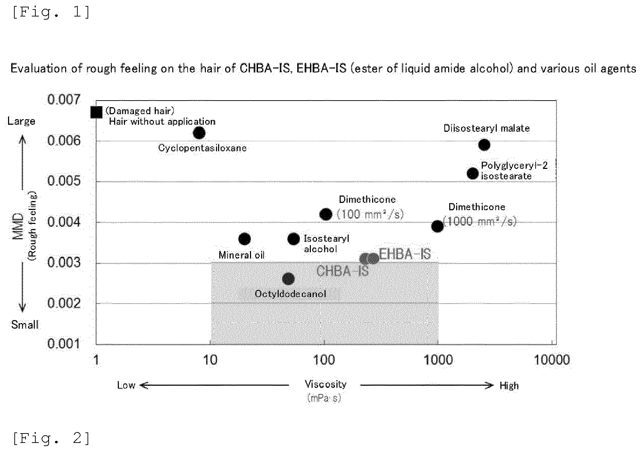

Figure 1 shows a diagram of measured values.

CHBA-IS has a viscosity of approximately 230 mPa·s and EHBA-IS has a viscosity of approximately 270 mPa·s. Generally, oils with high viscosity on the hair have a tendency to have a rough feeling (the tendency showed by gray arrow); however, CHBA-IS and EHAB-IS exhibit an effect to reduce rough feeling on the hair.

For example, compared with mineral oil, isostearyl alcohol and dimethicone (100 mm²/s) which have low viscosity, the rough feeling on the hair is low although the viscosity is high.

### <Pigment dispersibility:>

1. Weigh 1.5 g of a pigment in mortar.
2. Gradually add an oil dropwise and knead with a pestle.
3. Check the weight of the oil at each point of WP and FP.
4. Calculate WP and FP per 100 g of the pigment.
WP: Wet point (the lower the wet point, the better the wettability of the pigment.)
FP: Flow point (the lower the flow point, the better the dispersibility of the pigment.)

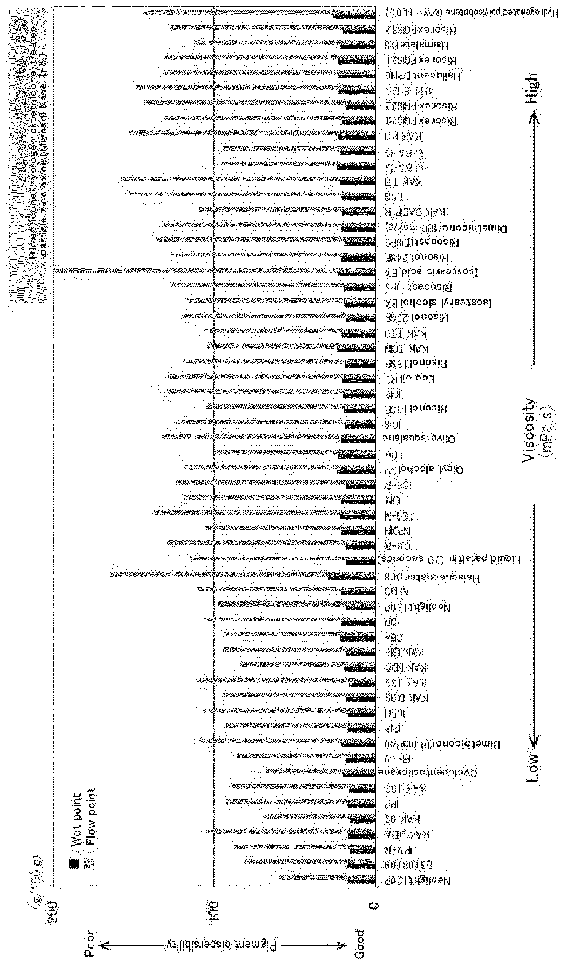

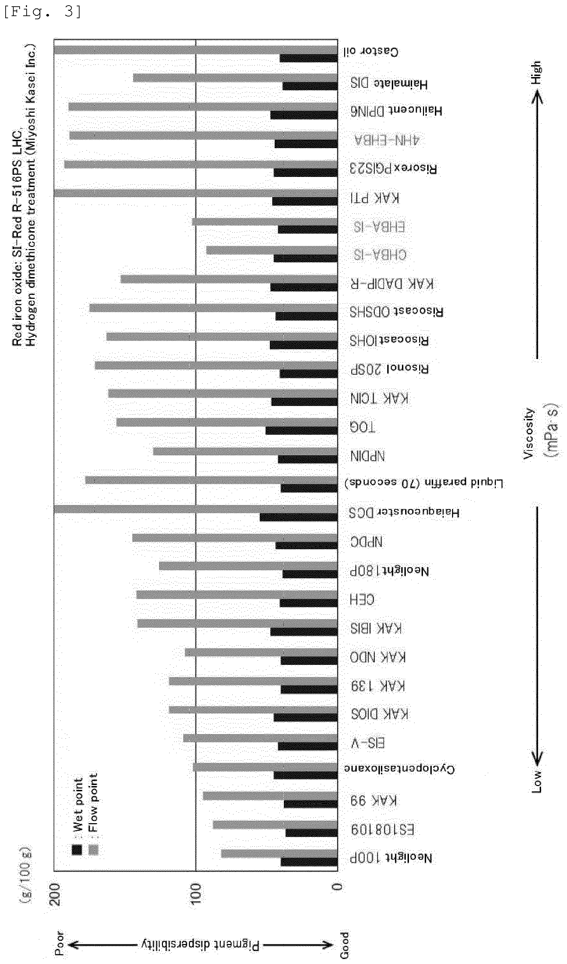

The dispersibility of zinc oxide is shown in Fig. 2, and the dispersibility of red iron oxide is shown in Fig. 3.

Regarding the pigment dispersibility of powder and the like, generally, the lower the viscosity, the better the dispersibility tends to be.

However, with respect to the ester of amide alcohol, it is understood that the dispersibility of the pigment is good although it has a relatively high viscosity. From this fact, in the formulation wherein a moist texture has been desired to be produced by originally using a high viscosity oil, but actually a low viscosity oil has been used because high viscosity oil has poor dispersibility, a possible formulation design of desired texture will expand because a large amount of pigment can be dispersed with a small amount of the oil agent.

The esters of amide alcohols which are liquid at room temperature (CHBA-IS, EHBA-IS) have excellent pigment dispersibility such as titanium oxide, zinc oxide, iron oxide, etc., even though the viscosity is high. That is, they have better powder dispersibility such as pigment than ester oils of the same viscosity. Therefore, they are suitable for use as a base for powder-containing cosmetic products (for example, UV care products, lipstick, foundation, cheek, eye shadow, etc.) and as a base for cleansing products.

### <Odor>

10 g of a sample was weighed to Maruemu screw tube No. 5, and left standing in an incubator at 45°C for 24 hours, and the odor was confirmed.

Confirmation of odor was carried out by 10 monitors, and judgment of odor was carried out based on the number of monitors judged as "having an odor". The results are shown in Table 2 below. No odor: 0 to 2 people judged as "having an odor"
Having a slight odor: 3 to 5 people judged as "having an odor" Having an odor: 5 to 10 people judged as "having an odor"

### [Table 2]

**Table 2: Evaluation of odor**

| | CHBA-IS | EHBA-IS | Eldew CL-202 | Eldew PS-203 |
|---|---|---|---|---|
| Odor (45°C 24hours) | No odor | No odor | Having an odor | Having a slight odor |

| | | | | |
|---|---|---|---|---|
| Eldew CL-202 (Ajinomoto): di(cholesteryl/octyldodecyl) lauroylglutamate Eldew PS-203 (Ajinomoto): di(phytosteryl/octyldodecyl) lauroylglutamate | | | | |

### [Industrial Applicability]

As mentioned above, the compound of formula (I) can be used in a variety of cosmetic formulations.

## Claims

1. A compound represented by formula (I): 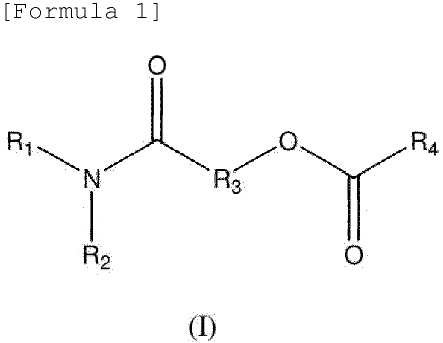 wherein
R₁ is an optionally substituted C6-C22 hydrocarbon group,
R₂ is H, or an optionally substituted C6-C22 hydrocarbon group,
R₃ is an optionally substituted linear or branched C2-C21 hydrocarbon group,
R₄ is an optionally substituted C5-C42 hydrocarbon group; or a group represented by formula (II) below: 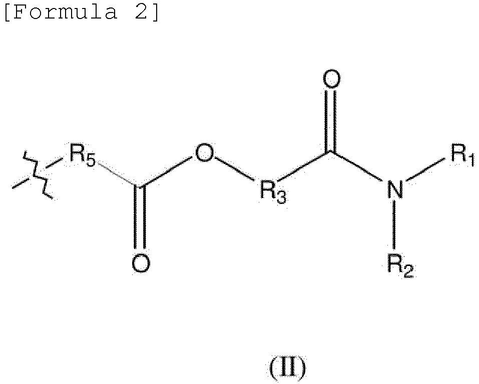
wherein in formula (II),
R₁ to R₃ are the same as defined above,
R₅ is an optionally substituted C2-C42 hydrocarbon group.

2. A thickener or a gelling agent comprising the compound according to Claim 1.

3. A powder dispersant comprising the compound according to Claim 1.

4. A texture improving agent comprising the compound according to Claim 1.

5. A cosmetic product comprising the compound according to Claim 1.

6. The cosmetic product according to Claim 5, comprising an oil agent.

7. The cosmetic product according to Claim 5 or 6, comprising a powder.

8. The cosmetic product according to Claim 5 or 6, which is for cleansing.

9. The cosmetic product according to any one of Claims 5 to 7, which is for make-up.
